# EUROPÄISCHE PATENTSCHRIFT

(11) **EP 2 029 037 B1**
(45) Veröffentlichungstag und Bekanntmachung des Hinweises auf die Patenterteilung: **23.10.2013**
(21) Anmeldenummer: 07729203.5
(22) Anmeldetag: 16.05.2007
(51) Int. Cl.: A61B 17/74, A61B 17/78, A61F 2/36, A61B 17/76, A61B 17/86, A61F 2/30

(54) **FEMURKOPF-IMPLANTAT**
FEMORAL HEAD IMPLANT
IMPLANT DE TÊTE FÉMORALE

(30) Priorität: 21.06.2006 DE 102006028449; 14.07.2006 DE 102006032811
(43) Veröffentlichungstag der Anmeldung: 04.03.2009
(73) Patentinhaber: Königsee Implantate und Instrumente zur Ostheosynthese GmbH, 07426 Königsee-Aschau (DE)
(72) Erfinder: ORSCHLER, Frank, 07426 Königsee/Aschau (DE); EHRHARDT, Arnd, 07426 Dörnfeld (DE); FINGER, Ulrich, 07318 Saalfeld (DE); MAIER, Klaus, 83043 Bad Aibling (DE)
(74) Vertreter: Meissner, Bolte & Partner
(86) Internationale Anmeldenummer: PCT/EP2007/054755
(87) Internationale Veröffentlichungsnummer: WO 2007/147688

(56) Entgegenhaltungen:
- WO-A-2005/025435
- FR-A2- 2 595 565
- US-A- 2 612 159
- US-A- 3 530 854
- US-A- 4 657 001
- US-A- 5 087 260
- US-A- 5 749 872
- US-A- 5 800 557

## Beschreibung

Die Erfindung betrifft ein Femurkopf-Implantat nach dem Oberbegriff des Anspruchs 1.

Zur Versorgung von Frakturen des Oberschenkelhalses bzw. zur Implantation künstlicher Femurköpfe sind aus dem Stand der Technik eine Reihe von Lösungen bekannt. Insbesondere sind dies singuläre Schrauben, Schrauben in Verbindung mit Gleitlaschen und eventuell zusätzlich eingebrachten Klingen in verschiedenen Varianten und proximale Femurnägel.

Singulare Schrauben dienen dabei sowohl als Stützschrauben für den versorgten Oberschenkelhals, als auch zu dessen Frakturkomprimierung. Dieser relativ starre Aufbau gewährleistet jedoch keine Dynamisierung der so ausgeführten Frakturbehandlung, d.h. dass das Implantat im wesentlichen keine Gleitmöglichkeit aufweist. Zudem weist ein derartiges Implantat vor allem in osteoporotischen Knochenstrukturen eine nur geringe Stabilität auf. Zudem ist bei der Implantation derartiger Schrauben ein mehr oder weniger streng paralleles Einbringen von mindestens zwei, zweckmäßigerweise aber drei getrennten Implantaten notwendig. Dies gestaltet sich in der chirurgischen Praxis mitunter als äußerst schwierig und ist oft nicht realisierbar.

Um diesem Problem zu begegnen, wurden nach dem Stand der Technik Implantate in Form von Tragschrauben in Verbindung mit Gleitlaschen vorgeschlagen. Ein derartiges Implantat wird beispielsweise in der deutschen Patentschrift DE 41 06 876 C2 beschrieben. Entsprechend der dort offenbarten Lehre wird eine Tragschraube proximal in Richtung des Hüftkopfes eingebracht. Die Tragschraube wird mit einer Lasche anschließend fixiert und geführt, Diese dient als extramedullärer Kraftträger und wird auf der Kortikalis befestigt. Der Schraubenschaft wird beim Sintern der Frakturzone teleskopisch geführt und ist gegen ein Verdrehen im dabei gebildeten Aufbau gesichert. Das Implantat ist damit dynamisierbar. Mittels einer Kompressionsschraube kann intraoperativ die Tragschraube in Richtung der Lasche gezogen und damit der Frakturspalt komprimiert werden.

Beim Eindrehen der Tragschraube in den Hüftkopf tritt ein Drehmoment auf, das den Hüftkopf versucht ebenfalls in Drehbewegung zu versetzen. Nach der Operation ist der Hüftkopf auf dem Schraubengewinde nicht hinreichend gegen Verdrehen gesichert. Bei der Belastung der Implantatzone tritt außerdem ein großes Kippmoment auf die Lasche auf. Um unter diesen Bedingungen eine ausreichende Stabilität der Osteosynthese im Frakturbereich zu gewährleisten, müssen in der Regel zusätzliche Implantate, zum Beispiel eine oder mehrere Antirotationsschrauben oder Trochanterabstützplatten bei pertrochanteren Frakturen, eingebracht werden. Dadurch erhöht sich der Invasionsgrad im Bereich des behandelten Knochenbereiches. Außerdem weisen derartige Implantate meist vorgegebene Winkelgeometrien auf, die mitunter zu einer individuellen Anatomie, insbesondere einem individuellen CCD-Winkel oder einer Varus-Valgus-Hüfte, nicht kompatibel gemacht werden können.

Bei den aus dem Stand der Technik bekannten Lösungen unter Verwendung proximaler Femur-Nägel, die beispielhaft in der Druckschrift DE 43 18 150 A1 offenbart ist, wird ein intramedullärer Kraftträger, in der Regel ein Nagel, proximal in den Markraum des Knochens eingebracht und distal verriegelt. Zur Aufnahme und Verriegelung des Hüftkopfes werden in den Nagel eine oder mehrere Schrauben bzw. Antirotationsstifte oder eine Klinge eingebracht. Dabei besteht der Nachteil, dass bei der Verwendung der Schraube immer eine zweite Schraube oder ein Bolzen zur Rotationssicherung des Frakturbereichs erforderlich ist. Bei osteoporotischen Knochenstrukturen haben Schrauben allein einen oft nur unzureichenden Halt. Bei einem schmalen Schenkelhals kann in Einzelfällen kein zweites Implantat zusätzlich zu der Tragschraube eingebracht werden.

Bei einer Verwendung einer Klinge, die durch Schläge vorgetrieben wird, besteht keine Kompressionsmöglichkeit bei der Behandlung von Schenkelhalsfrakturen. Vielmehr wird das Kopf-Halsfragment im Bereich der Fraktur beim Eindringen der Klinge auseinandergetrieben und es besteht die Gefahr einer bleibenden Vergrößerung des Frakturspaltes.

Zudem ist die Kombination mit einem Marknagel sehr invasiv, weil drei Zugänge durch das Weichteilgewebe eingebracht werden müssen.

Zum Stand der Technik sei noch auf die US 3,530,854, US 4,657,001, US 2,682,265 und US 2,612,159 aufmerksam gemacht. Bei diesen Druckschriften sind Femurkopf-Implantate gezeigt, die aus einer Anordnung aus Kraftträger, im Kraftträger gelagerten Anker sowie innerhalb des Ankers befindlichen, in Richtung des Femurkopfes vortreibbaren Tragschraube bestehen. Die Grundkonfiguration der vorbekannten Femurkopf-Implantate geht von einer Rotationssymmetrie, insbesondere in der Ausführungsform des Kraftträgers und dem gleitend gelagerten Anker aus. Rotationssymmetrische Anordnungen sind jedoch nicht ohne weiteres gegen Verdrehen stabil und erfordern aus Festigkeitsgründen recht große Durchmesser, so dass minimalinvasive Operationstechniken nicht möglich sind.

Aus der FR 2 595 565 A2 ist ein Hüftgelenk-Implantat vorbekannt, wobei eine Verlängerung eines Gelenkstücks in eine Hülsenlasche einsteckbar ist und bei der durch die Wahl der Querschnittsform eine Verdrehsicherheit gewährleistet ist.

Die gattungsbildende WO 2005/025435 A1 zeigt eine Vorrichtung zur Behandlung von Frakturen des Femur mit einem intramedullären Nagel mit zentraler Längsachse und einem den Hinterteil des Nagels schräg zur Längsachse durchbohrenden Durchgang mit unrundem Querschnitt. Die diesbezügliche Vorrichtung soll während der Implantation aufwendige Justiervorgänge für den Operateur entbehrlich machen und eine einfache blockier- und deblockierbare, formschlüssige Rotationsblockierung zwischen dem longitudinalen Knochenfixationselement und dem Marknagel erlauben.

Es ergibt sich aus dem Genannten die Aufgabe, ein Femurkopf-Implantat anzugeben, bei dem minivalinvasiv und kopferhaltend eine rotationsstabile und gleichzeitig kompressionsfähige Frakturbehandlung möglich ist. Optional soll darüber hinaus auch eine Möglichkeit geschaffen werden, im Falle einer Hüftkopfnekrose einen künstlichen Femurkopfersatz einzusetzen. Schließlich soll das geforderte Implantat-System auch für eine Korrekturosteotomie verwendbar sein.

Die Aufgabe wird mit einem Femurkopf-Implantat mit den Merkmalen des Anspruchs 1 gelöst, wobei die Unteransprüche zweckmäßige bzw. vorteilhafte Ausführungsformen des erfindungsgemäßen Gegenstandes beinhalten.

Das Fermurkopf-Implantat zeichnet sich durch eine Anordnung aus einem im wesentlichen in Richtung der Oberschenkelhals-Achse orientierten Kraftträger, einen innerhalb des Kraftträgers in dessen Längsrichtung gleitend gelagerten Anker und einer innerhalb des Ankers gleitend gelagerten, in Richtung der Femurkopf-Achse eingeschraubten Tragschraube aus.

In dem Femurkopf-Implantat ist somit die gleitende Verschiebbarkeit zwischen Tragschraube und Anker, sowie zwischen Anker und Kraftträger mit der durch die Tragschraube bewirkten Kompressionsmöglichkeit und der durch den Formschluss zwischen Anker und Kraftträger bewirkten Rotationssicherung vereinigt. Der Anker übernimmt dabei in Verbindung mit dem Kraftträger die bekannte Funktion der rotationsgesicherten Klinge, während die Tragschraube in Verbindung mit dem Anker und einem zusätzlichen Instrument intraoperativ die Kompressionsmöglichkeit des Frakturspaltes gewährleistet.

Durch die Ausrichtung aller Teile des Implantates in die Richtung der Schenkelhals-Achse und eine erfindungsgemäße Rechteckquerschnittsform von Kraftträger und Anker ist bei der Einbringung des Implantates nur ein einziger, verhältnismäßig kleiner Zugang für eine kopferhaltende Osteosynthese der Schenkelhalsfraktur unter weitgehender Schonung der Weichteile möglich. Es kann eine einzelne Tragschraube mit einem verhältnismäßig großen Durchmesser anstelle mehrerer Befestigungsmittel verwendet werden, wobei der Zugang zum Implantationsgebiet auf eine minimalinvasive Weise erfolgt.

In einer Ausführungsform weist die Tragschraube einen Gewindeabschnitt und einen zylindrischen Tragschraubenschaft mit einer axialen Längsbohrung und mindestens einer im Bereich des Gewindeabschnitts angeordneten Querbohrung auf. Der Tragschraubenschaft bildet dabei den im Anker gleitfähigen Teil der Schraube, während der Gewindeabschnitt in das Knochengewebe hineingedreht wird. Die Querbohrungen dienen in Verbindung mit der Längsbohrung vor allem als Drainageöffnungen.

Der zylindrische Tragschraubenschaft weist zweckmäßigerweise einen distalen Innensechskant zur Aufnahme eines Drehwerkzeuges auf. Zusätzlich dazu weist der zylindrische Tragschraubenschaft ein Innengewinde zum Eindrehen einer Kompressionsschraube bzw. eines Kompressionsinstrumentes auf.

Erfindungsgemäß ist der Kraftträger als ein Gleitkasten mit einer Gleitkastenaufnahme und einer extramedullären Befestigungslasche ausgebildet. Die Gleitkastenaufnahme dient der gleitenden Aufnahme des Ankers, die Befestigungslasche der extramedullären Befestigung des Gleitkastens.

Die Befestigungslasche weist dabei mindestens eine Bohrung zum Einbringen eines Befestigungsmittels, insbesondere einer Knochenschraube, auf.

Zusätzlich dazu ist die Befestigungslasche erfindungsgemäß mit mindestens einer Sollbiegestelle für intraoperatives Anformen versehen. Dadurch ist die Befestigungslasche auf die individuell vorliegende Femurform anmodellierbar.

Für extramedulläre Osteosynthesen und/oder Umstellungsosteotomien bei hüftgelenksnahem Frakturen und/oder Fehlstellungen weist die Befestigungslasche eine dafür erforderliche vergrößerte Länge auf.

Der Gleitkasten ist bei einer vorteilhaften erfindungsgemäßen Ausgestaltung mit mindestens einer, vorzugsweise zwei Führungsausformungen, insbesondere einer, vorzugsweise zwei Führungsrinnen oder Führungsbohrungen, zum Einführen mindestens eines Führungsdrahtes vorgesehen. Der Gleitkasten kann dadurch genau positioniert werden.

Bei einer weiteren Ausführungsform ist die Aufnahme des Kraftträgers als ein intramedullärer Nagel ausgebildet.

Der Anker weist bei einer Ausführungsform einen distalen Gleitschaft und einen proximalen verbreiterten Spitzenabschnitt mit einer durch den Gleitschaft verlaufenden Bohrung zur Aufnahme des Tragschraubenschaftes auf. Der distale Gleitschaft ist für eine gleitende Bewegung im Kraftträger, insbesondere im Gleitkasten, vorgesehen, während die Bohrung im Gleitschaft den Tragschraubenschaft gleitend aufnimmt.

Der Spitzenabschnitt weist mindestens eine mit der Führungsausformung am Gleitkasten fluchtende Ankerführung, insbesondere mindestens eine Ankerführungsrinne, auf. Dadurch können beim Einsetzen des Implantates Gleitkasten und Anker genau positioniert werden.

Zusätzlich kann eine den Gleitweg des Ankers verkürzende, insbesondere aufhebende, Blockiervorrichtung zum Einschieben und Befestigen in den Kraftträger, insbesondere den Gleitkasten, vorgesehen sein. Dadurch wird die Dynamisierbarkeit des Implantates falls erforderlich aufgehoben.

Die Blockiervorrichtung ist bei einer Ausführungsform als eine stempelförmige Verlängerung mit einem Blockierungsplättchen ausgebildet. Das Blockierungsplättchen ist zweckmäßigerweise an der extramedullären Befestigungslasche arretierbar, insbesondere mit einem Befestigungsmittel fixierbar.

Weiterhin kann bei einer zweckmäßigen Ausführungsform der Anker in Form eines Hüftkopfadapters ausgebildet sein, wobei dieser im Bereich des Spitzenabschnitts einen Hüftkopfabschnitt aufweist. In diesem Fall ist zwischen dem Hüftkopfabschnitt und dem Gleitschaft ein Kragenabschnitt für eine abstützende Lagerung des Hüftkopfadapters auf dem Kraftträger vorgesehen.

Das Femurkopf-Implantat weist in einer vorteilhaften erfindungsgemäßen Ausgestaltung zusätzlich auf der das Knochengewebe berührenden Oberfläche des Gleitkastens eine osteophile Struktur und/oder eine osteophile und/oder antibiotische Beschichtung auf. Diese verbessert die Osteosynthese in der unmittelbaren Umgebung des Implantates.

Weiterhin weist der Gewindeabschnitt der Tragschraube mindestens eine Querbohrung in Verbindung mit einer innerhalb der Tragschraube verlaufenden axialen Längsbohrung aufweist. Dadurch wird ein Drainagekanal zum Ableiten von sich im Hüftkopf ansammelnder Flüssigkeit gebildet.

Der Gewindeabschnitt der Tragschraube weist an dessen Spitze mindestens eine Einfräsung zur Ausbildung einer Selbstschneide auf. Hierdurch wird der Vortrieb in das Knochengewebe erleichtert, wobei das Knochengewinde durch den Gewindekopf eingeschnitten wird.

Das Femurkopf-Implantat soll im Folgenden anhand von Ausführungsbeispielen näher erläutert werden. Zur Verdeutlichung dienen die Figuren 1 bis 8. Für gleiche oder gleich wirkende Teile werden die selben Bezugszeichen verwendet.

Es zeigt:
Fig. 1 eine beispielhafte Anordnung aus Tragschraube, Anker und Gleitkasten mit einer extramedullären Befestigung am Femurschaft,
Fig. 2a eine erste Ansicht der Tragschraube,
Fig. 2b eine zweite Ansicht der Tragschraube mit einer Darstellung des Innensechskants,
Fig. 3a eine Seitenansicht des Gleitkastens,
Fig. 3b eine Ansicht des Gleitkastens mit einer Darstellung der Gleitkastenaufnahme und der Sollbiegestelle der Befestigungslasche,
Fig. 4a eine Seitenansicht des Ankers mit Gleitschaft und Spitze,
Fig. 4b einen beispielhaften Führungsdraht,
Fig. 5a eine beispielhafte Blockiervorrichtung zur Gleitwegverkürzung bzw.-aufhebung,
Fig. 5b eine Darstellung eines Hüftkopfadapters und dessen Zusammenbau mit dem Gleitkasten,
Fig. 6 eine Ansicht des Femurkopfimplantats in einer extramedullären Position,
Fig. 7 eine Ansicht des Hüftkopfadapters mit Gleitkasten aus Fig. 5b in einer extramedullären Position,
Fig. 8 eine Ansicht des Femurkopfimplantats in Verbindung mit einem Marknagel zur intramedullären Kraftaufnahme.

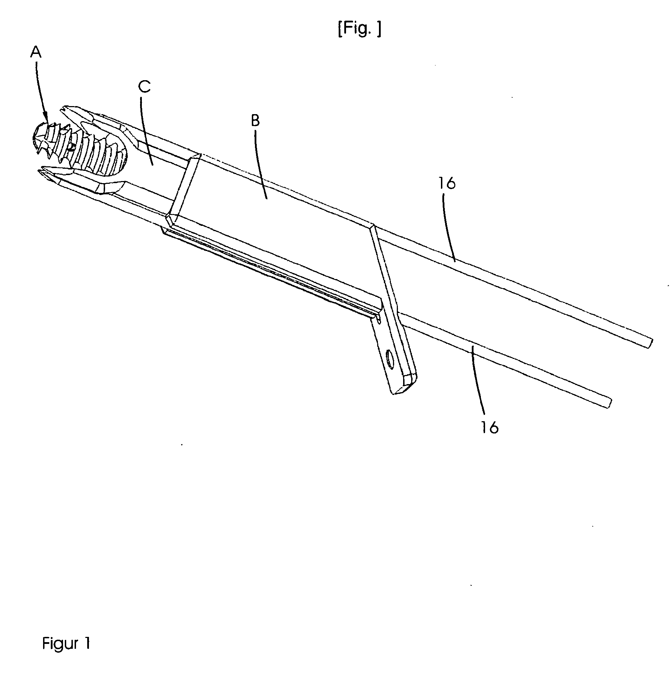

Fig. 1 zeigt ein beispielhaftes Femurkopf-Implantat. Das Implantat besteht aus einer Tragschraube A, einem rechteckförmigen Gleitkasten B und einem ebenfalls im Querschnitt stab- oder rechteckförmigen Anker C, die ineinander in Richtung der Längsachse der gesamten Anordnung mit einem geringen Spiel gleitend gelagert sind. Zur einfachen Positionierung des Implantats sind Führungsdrähte 16 vorgesehen. Im Gleitkasten ist eine rechteckförmige Durchgangsöffnung angebracht, welche auf die Außenkontur des Ankers abgestimmt ist.

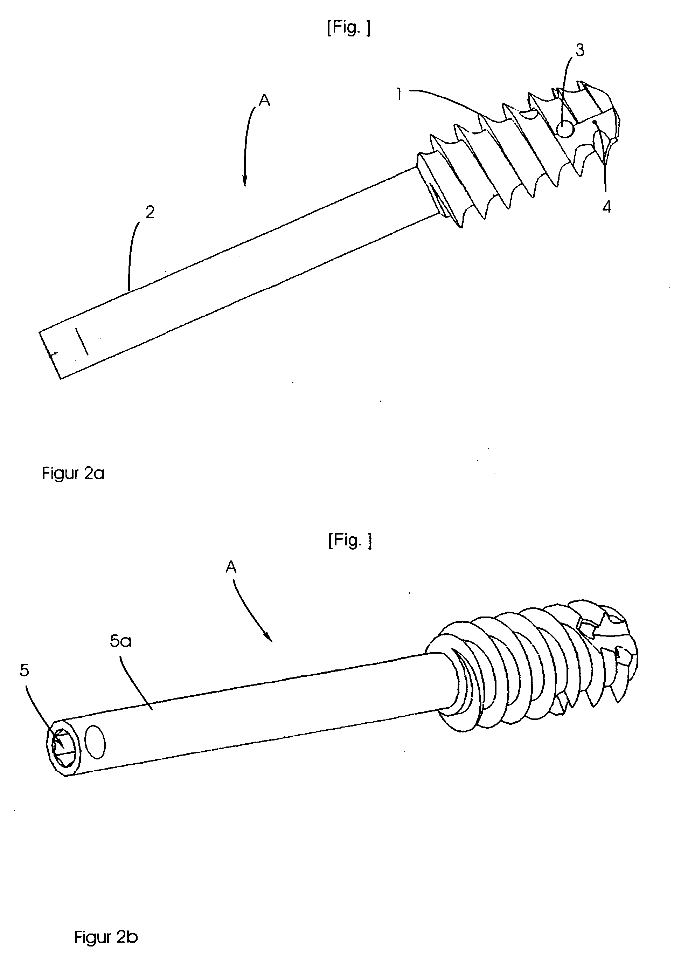

Die Fig. 2a und 2b zeigen die Tragschraube in Einzelansichten. Die Tragschraube weist einen Gewindeabschnitt 1 und einen sich daran anschließenden Tragschraubenschaft 2 auf. Der Tragschraubenschaft ist im wesentlichen zylindrisch geformt. Der Gewindedurchmesser beträgt etwa 7 bis 13, vorzugsweise 10 bis 12 Millimeter, während die Gewindelänge etwa 10 bis 30, vorzugsweise 15 bis 25 Millimeter beträgt.

Die Tragschraube weist in ihrer gesamten Länge eine durchgehende Längsbohrung auf. Diese ist im Bereich des Gewindeabschnittes mit mindestens einer Querbohrung 3 verbunden und bildet eine Drainageeinrichtung zur Druckminderung im Hüftkopf aus. Damit wird das Risiko einer Hüftkopfnekrose, insbesondere bei Schenkelhalsfrakturen, minimiert und ein Druckabbau bei Ansammlung von Flüssigkeit im Hüftkopf bewirkt.

Durch eine oder mehrere von Einfräsungen 4 auf dem Gewinde ist eine Selbstschneide für das Knochengewinde ausgebildet. Zum Eingreifen eines Drehwerkzeugs beim Vorschrauben der Tragschraube dient ein am anderen Ende des Tragschraubenschaftes angeordneter Innensechskant 5. Zum Eindrehen einer Kompressionsschraube oder eines anderen Kompressionsinstrumentes ist in der Längsbohrung innerhalb des Tragschraubenschaftes hinter dem Innensechskant ein hier verdecktes Innengewinde 5a vorgesehen.

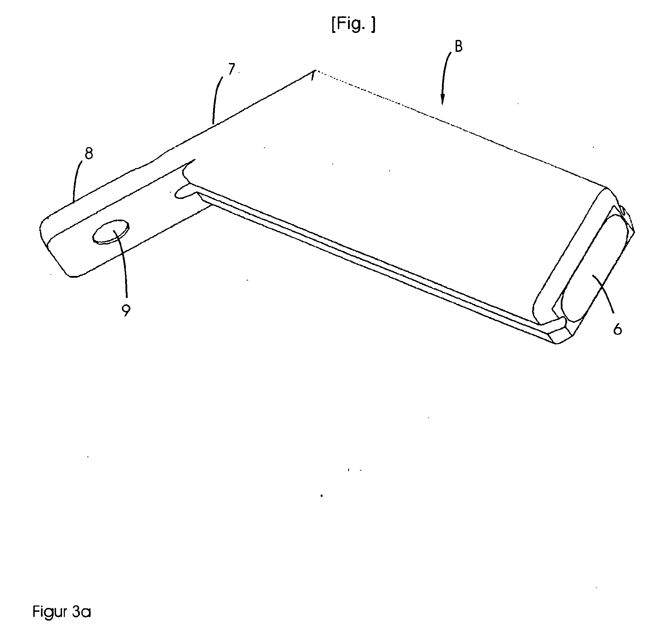

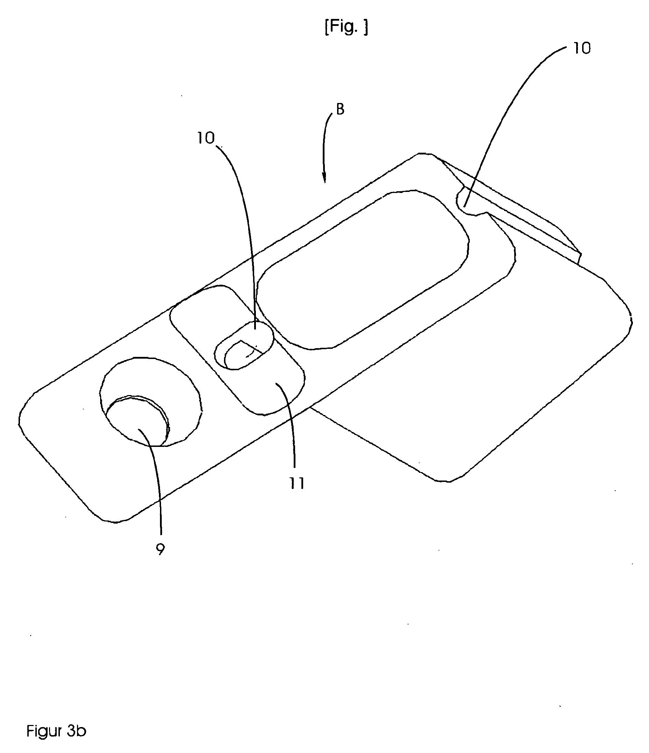

Die Figuren 3a und 3b zeigen den Gleitkasten B in verschiedenen Ansichten. Der Gleitkasten weist eine rechteckförmige, mit Rundungsfasen in den Eckbereichen versehene Ankeraufnahme 6 zur gleitenden Führung des Ankers C auf. Er weist am distalen Ende eine anatomiegerechte Abschrägung 7 auf. Zusätzlich ist eine Befestigungslasche 8 mit einer Bohrung 9 vorgesehen, durch die Befestigungsmittel, beispielsweise Knochenschrauben, diese die Kortikalis in den Knochen eingebracht werden können.

Der Gleitkasten weist je eine an den Rechteck-Schmalseiten außerhalb des Ankeraufnahmedurchbruchs angeordnete Führungsrinne 10 zur Aufnahme je eines Führungsdrahtes 16 auf. Außerdem ist die Befestigungslasche 8 über eine Sollbiegestelle 11 intraoperativ an die individuell vorliegende Knochenform anformbar. Für eine verbessere Osteointegration kann die im Knochen befindliche Oberfläche des Gleitkastens mit einer osteophilen Struktur versehen und/oder mit osteophilen und/oder antibiotischen Substanzen beschichtet sein.

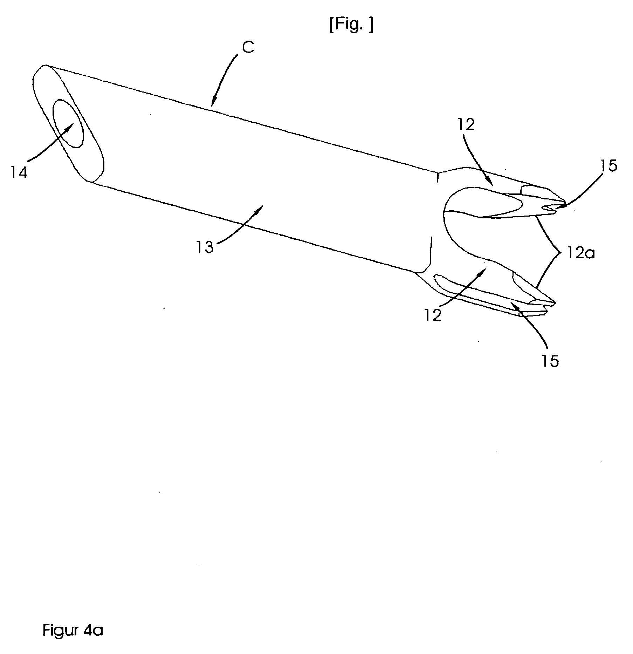

Fig. 4a zeigt einen beispielhaften Anker C. Der Anker besteht aus einem Spitzenabschnitt 12 mit Schneiden 12a und einem Gleitschaft 13. Der Gleitschaft ist so bemessen, dass er in der Ankeraufnahme 6 des Gleitkastens mit einem geringen Spiel gleitfähig gelagert ist. Eine Ankerbohrung 14 dient einer gleitenden Lagerung des Tragschraubenschaftes 2. Die Gleitschaftaußenkontur entspricht dabei der im Wesentlichen rechteckigen, mit Rundungsfasen versehenen Gestalt der Ankeraufnahme 6.

Der U-förmige, als Gabel ausgebildete Spitzenabschnitt 12 weist zwei Führungsrinnen 15 auf, die der Aufnahme je eines Führungsdrahtes 16 dienen. Diese liegen in Flucht mit den Führungsrinnen 10 des Gleitkastens und befinden sich an den Außenseiten der Gabel.

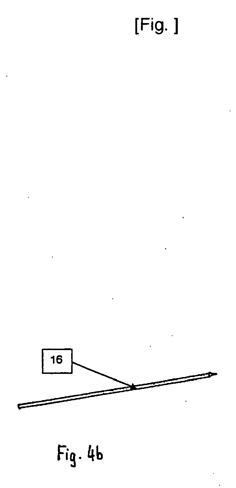

Fig. 4b zeigt einen beispielhaften Führungsdraht. Dieser dient zur intraoperativen Führung von Gleitkasten und Anker sowie einer Retention des Hüftkopf-Fragmentes nach einer erfolgten geschlossenen Reposition auf einem Extensionstisch.

Bei einer Verwendung von zwei Führungsdrähten wird zu Beginn des Implantationseingriffs die Lage von Gleitkasten und Anker in axialer und ap-Lage festgelegt und eine sichere und standardisierte Operationstechnik gewährleistet. Hierzu wird über Bohrschablonen der zukünftige Implantatsitz vorgebohrt. Tragschraube A, Gleitkasten B und Anker C werden gemäß Fig. 1 ineinander geschoben, wobei die Tragschraube an ihrem Tragschraubenschaft in die Ankerbohrung so eingesetzt wird, dass sich der Gewindeabschnitt der Tragschraube im Bereich des Spitzenabschnitts des Ankers befindet. Anschließend wird der Anker über dessen Gleitschaft in die Ankeraufnahme des Gleitkastens so eingeschoben, dass sich der Spitzenabschnitt des Ankers auf dem der Befestigungslasche des Gleitkastens entgegen gesetzten Ende des Gleitkastens befindet und die abgeschrägten Enden von Anker und Gleitkasten parallel verlaufen.

Die Führungsdrähte werden selbstbohrend über eine Bohrschablone eingesetzt und das Implantat wird entlang der erwähnten Führungsrinnen in den Knochen eingebracht. Dann wird die Tragschraube so weit wie möglich in den Femurkopf eingedreht und anschließend der Anker bis zum Anschlag so weit vorgetrieben, dass er auf dem Gewinde aufsitzt. Anschließend werden die Tragschraube und der Anker gemeinsam zurück gezogen und komprimieren dabei die Frakturzone. Hierzu wird eine Kompressionsschraube oder ein Kompressionsinstrument in das Innengewinde des Tragschraubenschaftes eingedreht und mit der Schraube das Kopffragment zurückgezogen.

Die Länge des Gewindeabschnitts der Tragschraube und die Länge des Spitzenabschnitts des Ankers sind so bemessen, dass beim Zurückziehen des Implantats intraoperativ bei der Frakturspaltkompression oder postoperativ beim Sintern der Fraktur beide Implantatteile gemeinsam in axialer Richtung gleiten. Das Gewinde der Tragschraube passt nicht durch die Ankerbohrung und nimmt somit den Anker bei dessen Gleitbewegung im Gleitkasten mit.

Bei den beschriebenen Ausführungsbeispielen können eine Reihe von Abänderungen vorgenommen werden. So kann der Gleitkasten mittels eines Nagels als Kraftträger für Tragschraube und Anker eingesetzt werden.

Die Befestigungslasche des Gleitkastens kann für extramedulläre Osteosynthesen bzw. Umstellungsosteotomien von hüftgelenksnahen Frakturen bzw. Fehlstellungen beliebig verlängert sein.

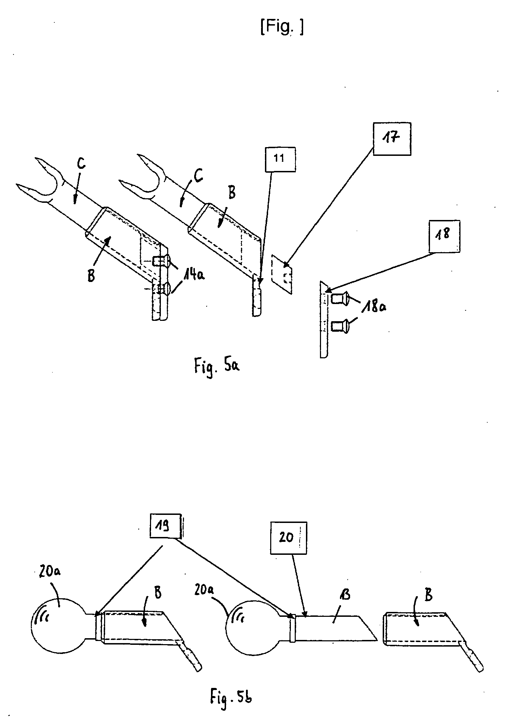

Besonders zweckmäßig für eine möglicherweise erforderliche Aufhebung der Dynamisierung sind gleitwegsvermindernde oder -aufhebende Vorrichtungen. Ein diesbezügliches Beispiel ist in Fig. 5a dargestellt. Eine der Form und Größe des Gleitschaftes 13 entsprechende stempelförmige Verlängerung 17 mit einem Blockierungsplättchen 18 wird in die laterale Öffnung der Ankeraufnahme eingeführt und stößt dabei gegen das abgeschrägte laterale Ende des Gleitschaftes des Ankers im Gleitkasten. Nach der Entfernung des kaudalen Führungsdrahtes 16 wird das Blockierungsplättchen mit je einer Schraube 18a in der Verlängerung 17 bzw. in einem Loch an der Sollbiegestelle 11 der Befestigungslasche fixiert.

Bei Bedarf, zum Beispiel zur extramedullären Versorgung einer instabiler Fraktur mit einer langen Befestigungslasche am Gleitkasten, kann das Blockierungsplättchen 18 auch ohne Verlängerung 17 aufgeschraubt werden und dabei die Sollbiegestelle 11 versteifen.

Weiterhin ist auch das Aufsetzen eines Hüftkopfadapters 20 möglich. Bei einer entsprechenden Indikation, z.B. bei einem geriatrischen Patienten mit einer als Komplikation nach der Versorgung der Oberschenkelhalsfraktur eintretenden Hüftkopfnekrose, kann durch einen zweiten minimalinvasiven Eingriff diese Komplikation durch einen Hüftkopfersatz behandelt und der wesentlich größere traumatisierendere Eingriff der Metallentfernung bei anschließendem endoprothetischen Ersatz vermieden werden. Fig. 5b zeigt ein diesbezügliches Beispiel.

Bei diesem Beispiel wird in den Gleitkasten B ein Hüftkopfadapter 20 eingebracht. Die so gebildete Anordnung dient als Hüftgelenkendoprothese. Der Adapter weist unterhalb des Kugelkopfes 20a einen umlaufenden Kragen 19 auf, der sich auf dem Gleitkasten abstützt.

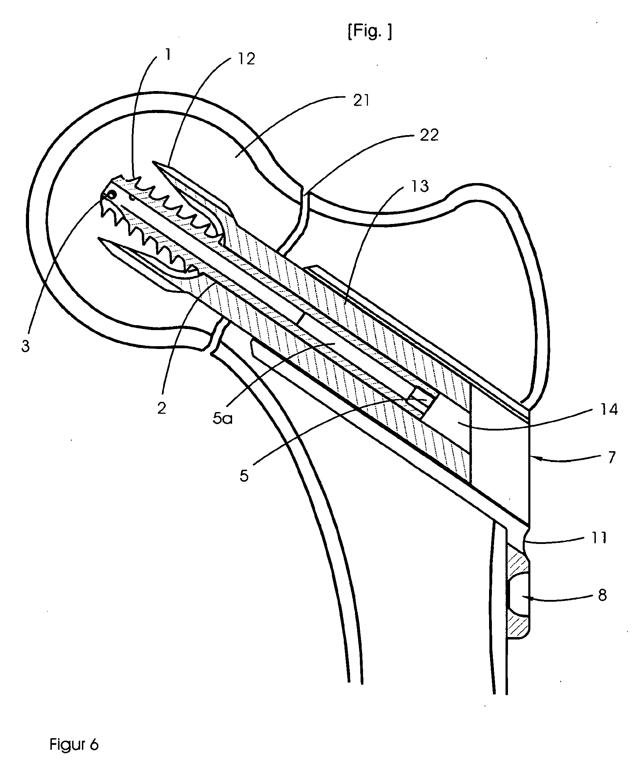

Fig. 6 zeigt das in den Figuren 1 bis 4a beschriebene Implantat in seiner Position in einem Hüftkopf 21 mit einer Frakturlinie 22. Zur erkennen ist, dass sich der Gewindeabschnitt 1 der Tragschraube A und der Spitzenabschnitt 12 des Ankers C mit seinen Schneiden 12a jenseits der Frakturlinie befindet. Der Gleitkasten B ist so eingeführt, dass durch die Abschrägung 7 die Befestigungslasche 8 flächig auf der Kortikalis aufliegt.

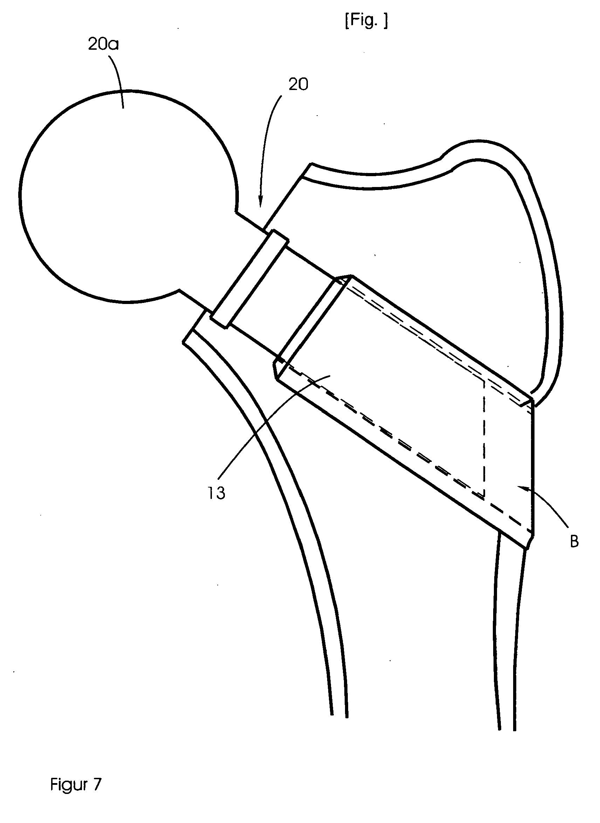

Fig. 7 zeigt den in Fig. 5b gezeigten Hüftkopfadapter 20 mit Gleitkasten B in seiner Position. Der Gleitkasten B ist hier entsprechend der Darstellung aus Fig. 6 eingeführt. Der ursprüngliche jenseits des Frakturspaltes in proximaler Richtung gelegene nekrotische Teil des Hüftkopfes wurde zusammen mit der ursprünglichen Tragschraube und dem ursprünglichen Anker entfernt. Anstelle des ursprünglichen Ankers aus Fig. 6 ist nun der gezeigte Hüftkopfadapter 20 mit einer künstlichen Hüftkugel 20a in den im Knochen verbliebenen Gleitkasten eingesetzt.

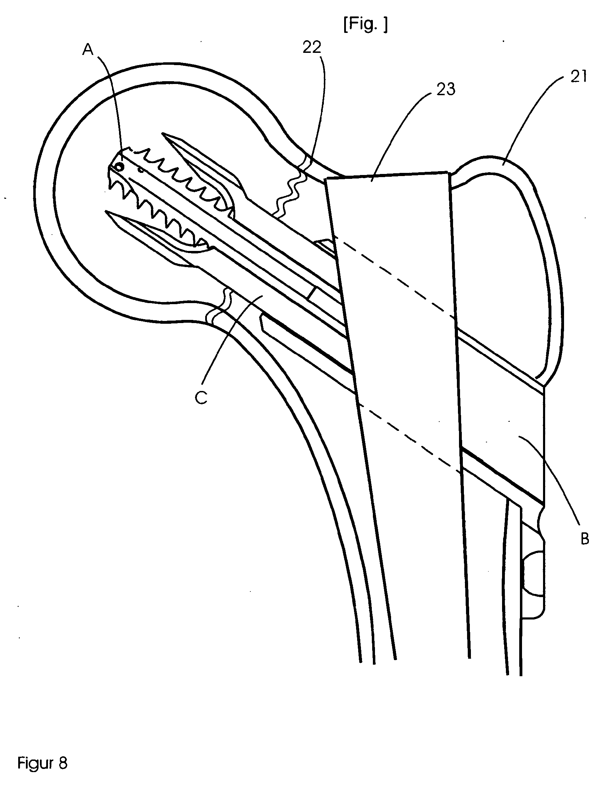

Fig. 8 zeigt eine Kombination des erfindungsgemäßen Implantatbeispiels aus den vorherigen Figuren mit einem Marknagel 23. Bei diesem Ausführungsbeispiel wird der Gleitkasten durch eine Öffnung des Marknagels hindurch in den Oberschenkelhals getrieben und im Marknagel stabilisiert und fixiert.

Weitere Ausführungsformen ergeben sich aus den Unteransprüche bzw. durch Ausgestaltungen der dargestellten Ausführungsbeispiele im Rahmen fachmännischen Handelns.

### Bezugszeichenliste

- A: Tragschraube
- 1: Gewindeabschnitt
- 2: Tragschraubenschaft
- 3: Querbohrung
- 4: Einfräsung
- 5: Innensechskant
- 5a: Innengewinde (verdeckt)

- B: Gleitkasten
- 6: Ankeraufnahme
- 7: Abschrägung
- 8: Befestigungslasche
- 9: Bohrung
- 10: Führungsrinne
- 11: Sollbiegestelle

- C: Anker
- 12: Spitzenabschnitt
- 12a: Schneide
- 13: Gleitschaft
- 14: Ankerbohrung
- 15: Führungsrinne am Anker
- 16: Führungsdraht

- 17: stempelförmige Verlängerung
- 18: Blockierungsplättchen
- 18a: Schraube
- 19: umlaufender Kragen
- 20: Hüftkopfadapter
- 20a: künstliche Hüftkugel
- 21: Hüftkopf
- 22: Frakturlinie
- 23: Marknagel

## Patentansprüche

1. Femurkopf-Implantat,
bestehend aus einer Anordnung aus einem in Richtung der Oberschenkelhals-Achse orientierten Kraftträger (B), einem innerhalb des Kraftträgers in dessen Längsrichtung gleitend gelagerten Anker (C) und einer innerhalb des Ankers gleitend gelagerten, in Richtung des Femurkopfes vorgetriebenen Tragschraube (A),
**dadurch gekennzeichnet, dass**
der Kraftträger als ein im Querschnitt rechteckförmiger Gleitkasten (B) mit einer rechteckförmigen Gleitkastenaufnahme (6) und einer extramedullären Befestigungslasche (8) ausgebildet ist, und daß der Anker (C) im Querschnitt rechteckförmig ist.

2. Femurkopf-Implantat nach Anspruch 1,
**dadurch gekennzeichnet, dass**
die Tragschraube (A) einen Gewindeabschnitt (1) und einen zylindrischen Tragschraubenschaft (2) mit einer axialen Längsbohrung und mindestens einer im Bereich des Gewindeabschnitts angeordneten Querbohrung (3) aufweist.

3. Femurkopf-Implantat nach Anspruch 2,
**dadurch kennzeichnet, dass**
der zylindrische Tragschraubenschaft (2) einen distalen Innensechskant (5) zum Einsetzen eines Drehwerkzeugs aufweist.

4. Femurkopf-Implantat nach einem der Ansprüche 2 oder 3,
**dadurch gekennzeichnet, dass**
der zylindrische Tragschraubenschaft (2) ein Innengewinde (5a) zum Eindrehen einer Kompressionsschraube oder eines -instrumentes aufweist.

5. Femurkopf-Implantat nach Anspruch 1,
**dadurch gekennzeichnet, dass**
die Befestigungslasche (8) mindestens eine Bohrung (9) zum Einbringen eines Befestigungsmittels, insbesondere einer Knochenschraube, aufweist.

6. Femurkopf-Implantat nach einem der Ansprüche 1 oder 5,
**dadurch gekennzeichnet, dass**
die Befestigungslasche (8) mindestens eine Sollbiegestelle (11) für ein intraoperatives Anformen aufweist.

7. Femurkopf-Implantat nach einem der Ansprüche 1 bis 6,
**dadurch gekennzeichnet, dass**
die Befestigungslasche (8) eine für Osteosynthesen bei Schenkelhalsfrakturen und/oder Umstellungsosteotomien bei Fehlstellungen erforderliche vergrößerte Länge aufweist.

8. Femurkopf-Implantat nach einem der Ansprüche 1 bis 7,
**dadurch gekennzeichnet, dass** der Gleitkasten (B) mindestens eine, vorzugsweise zwei Führungsausformungen, insbesondere eine, vorzugsweise zwei Führungsrinnen (10) oder Führungsbohrungen, zum Einführen mindestens eines Führungsdrahtes (16) an seinen Längsschmal-Außenseiten aufweist.

9. Femurkopf-Implantat nach Anspruch 1,
**dadurch gekennzeichnet, dass**
der Anker (C) einen distalen Gleitschaft (13) und einen proximalen verbreiterten gabelförmigen Spitzenabschnitt (12) mit einer durch den Gleitschaft verlaufenden Bohrung (14) zur gleitenden Aufnahme des Tragschraubenschaftes (2) aufweist.

10. Femurkopf-Implantat nach Anspruch 8 und 9,
**dadurch gekennzeichnet, dass**
der gabelförmige Spitzenabschnitt (12) mindestens eine, vorzugsweise zwei, mit der Führungsausformung am Gleitkasten fluchtende außenliegende Ankerführungen, insbesondere mindestens eine Ankerführungsrinne (15) aufweist.

11. Femurkopf-Implantat nach Anspruch 1,
**dadurch gekennzeichnet, dass**
eine den Gleitweg des Ankers (C) verkürzende, insbesondere aufhebende, Blockiervorrichtung zum Einschieben und Befestigen in den Gleitkasten (B), vorgesehen ist.

12. Femurkopf-Implantat nach einem der vorhergehenden Ansprüche,
**dadurch gekennzeichnet, dass**
der Gewindeabschnitt (1) der Tragschraube (A) mindestens eine Querbohrung (3) in Verbindung mit einer innerhalb der Tragschraube verlaufenden axialen Längsbohrung aufweist.

13. Femurkopf-Implantat nach einem der vorhergehenden Ansprüche,
**dadurch gekennzeichnet, dass**
der Gewindeabschnitt (1) der Tragschraube (A) an dessen Spitze mindestens eine Einfräsung (4) zur Ausbildung einer Selbstschneide aufweist.

14. Femurkopf-Implantat nach einem der vorangegangenen Ansprüche,
**dadurch gekennzeichnet, dass**
die Gleitkastenaufnahme (6) Rundungsfasen in den Eckbereichen aufweist.

## Claims

1. Femoral head implant consisting of an arrangement of a load carrier (B) oriented in the direction of the femoral neck axis, an anchor (C) mounted in a sliding manner inside the load carrier in the longitudinal direction thereof, and a carrier screw (A) mounted in a sliding manner inside the anchor and driven in the direction of the femoral head,
**characterized in that**
the load carrier is designed as a slide box (B) with a rectangular cross-section, having a rectangular slide box receptacle (6) and an extra-medullary fixing tab (8), and that the anchor (C) has a rectangular cross-section.

2. Femoral head implant according to claim 1,
**characterized in that**
the carrier screw (A) comprises a threaded portion (1) and a cylindrical carrier screw shaft (2) having an axial longitudinal hole and at least one cross hole (3) arranged in the area of the threaded portion.

3. Femoral head implant according to claim 2,
**characterized in that**
the cylindrical carrier screw shaft (2) comprises a distal hexagon socket (5) for the insertion of a turning tool.

4. Femoral head implant according to one of claims 2 or 3,
**characterized in that**
the cylindrical carrier screw shaft (2) comprises an internal thread (5a) for screwing in a compression screw or compression instrument.

5. Femoral head implant according to claim 1,
**characterized in that**
the fixing tab (8) comprises at least one bore (9) for inserting a fixing means, in particular a bone screw.

6. Femoral head implant according to one of claims 1 or 5,
**characterized in that**
the fixing tab (8) comprises at least one predetermined bending point (11) for an inoperative adaptation.

7. Femoral head implant according to one of claims 1 to 6,
**characterized in that**
the fixing tab (8) comprises an increased length required for osteosyntheses in the case of fractures of the femoral neck and/or corrective osteotomies in the case of malpositions.

8. Femoral head implant according to one of claims 1 to 7,
**characterized in that**
the slide box (B) comprises at least one, preferably two molded guiding portions, in particular one, preferably two guiding grooves (10) or guiding holes for inserting at least one guiding wire (16) on the longitudinal narrow outer sides thereof.

9. Femoral head implant according to claim 1,
**characterized in that**
the anchor (C) comprises a distal sliding shaft (13) and a proximal, widened fork-shaped point part (12) including a hole (14) extending through the sliding shaft for receiving in a sliding manner the carrier screw shaft (2).

10. Femoral head implant according to claim 8 and 9,
**characterized in that**
the fork-shaped point part (12) comprises at least one, preferably two external anchor guides aligned with the molded guiding portion on the slide box, in particular at least one anchor guiding groove (15).

11. Femoral head implant according to claim 1,
**characterized in that**
an inhibiting device shortening, in particular canceling the sliding distance of the anchor (C) is provided for insertion into and fixing in the slide box (B).

12. Femoral head implant according to one of the preceding claims,
**characterized in that**
the threaded portion (1) of the carrier screw (A) comprises at least one cross hole (3) in connection with an axial longitudinal hole extending inside the carrier screw.

13. Femoral head implant according to one of the preceding claims,
**characterized in that**
the threaded portion (1) of the carrier screw (A) comprises at the point thereof at least one milled portion (4) to create a self-cutting edge.

14. Femoral head implant according to one of the preceding claims,
**characterized in that**
the slide box receptacle (6) comprises rounded chamfers in the corner regions.

## Revendications

1. Implant de tête fémorale, constitué d'un agencement composé d'un élément d'encaissement de force (B) orienté en direction de l'axe du col du fémur, d'un ancrage (C) monté à l'intérieur de l'élément d'encaissement de force en coulissement dans sa direction longitudinale, et d'une vis portante (A) montée en coulissement à l'intérieur de l'ancrage et forcée en direction de la tête du fémur,
**caractérisé en ce que**
l'élément d'encaissement de force est réalisé sous la forme d'un caisson coulissant (B) à section de forme rectangulaire avec un logement de caisson coulissant (6) de forme rectangulaire et une patte de fixation (8) extra-médullaire, et **en ce que** l'ancrage (C) a une section de forme rectangulaire.

2. Implant de tête fémorale selon la revendication 1,
**caractérisé en ce que** la vis portante (A) comporte un tronçon à pas de vis (1) et une tige de vis cylindrique (2) avec un perçage longitudinal axial et avec au moins un perçage transversal (3) agencé dans la région du tronçon à pas de vis.

3. Implant de tête fémorale selon la revendication 2,
**caractérisé en ce que** la tige de vis cylindrique (2) comporte une empreinte hexagonale intérieure distale (5) pour introduire un outil tournant.

4. Implant de tête fémorale selon l'une des revendications 2 ou 3,
**caractérisé en ce que** la tige de vis cylindrique comporte un pas de vis intérieur (5a) pour visser une vis de compression ou un instrument de compression.

5. Implant de tête fémorale selon la revendication 1,
**caractérisé en ce que** la patte de fixation (8) comporte au moins un perçage (9) pour l'introduction d'un organe de fixation, en particulier d'une vis à os.

6. Implant de tête fémorale selon l'une des revendications 6 ou 5,
**caractérisé en ce que** la patte de fixation comporte au moins un emplacement de flexion de consigne (11) pour une mise en forme intra-opératoire.

7. Implant de tête fémorale selon l'une des revendications 1 à 6,
**caractérisé en ce que** la patte de fixation (8) présente une longueur augmentée nécessaire pour des ostéosynthèses en cas de fracture du col du fémur et/ou des ostéotomies de remise en place dans le cas d'anomalies de position.

8. Implant de tête fémorale selon l'une des revendications 1 à 7,
**caractérisé en ce que** le caisson coulissant (B) comporte au moins une et de préférence deux conformations de guidage, en particulier un(e) et de préférence deux rainure(s) de guidage (10) ou perçage(s) de guidage, pour l'introduction d'au moins un fil de guidage (16) au niveau de ses faces extérieures longitudinales étroites.

9. Implant de tête fémorale selon la revendication 1,
**caractérisé en ce que** l'ancrage comprend une tige coulissante distale (13) et un tronçon en pointe proximal (12) qui s'élargit en forme de fourche, avec un perçage (14) s'étendant à travers la tige coulissante pour recevoir en coulissement la tige de la vis portante (2).

10. Implant de tête fémorale selon la revendication 8 et 9,
**caractérisé en ce que** le tronçon de pointe en forme de fourche (12) comprend au moins un et de préférence deux guidage(s) d'ancrage disposé(s) à l'extérieur et en alignement avec la conformation de guidage sur le caisson coulissant, en particulier au moins une rainure de guidage (15) pour l'ancrage.

11. Implant de tête fémorale selon la revendication 1,
**caractérisé en ce qu'**il est prévu un dispositif de blocage qui raccourcit le trajet de glissement de l'ancrage (C), en particulier qui annule ce trajet, pour l'introduction et la fixation dans le caisson coulissant (B).

12. Implant de tête fémorale selon l'une des revendications précédentes,
**caractérisé en ce que** le tronçon à pas de vis (1) de la vis portante (A) comporte au moins un perçage transversal (3) en communication avec un perçage longitudinal axial s'étendant à l'intérieur de la vis portante.

13. Implant de tête fémorale selon l'une des revendications précédentes,
**caractérisé en ce que** le tronçon à pas de vis (1) de la vis portante (A) comporte au niveau de sa pointe au moins un fraisage (4) pour réaliser un élément auto-tailleur.

14. Implant de tête fémorale selon l'une des revendications précédentes,
**caractérisé en ce que** le logement de caisson coulissant (6) comporte des congés arrondis dans les zones de coin.
